(19) Europäisches Patentamt
European Patent Office
Office européen des brevets

(11) Publication number: 0 026 811 B1

(12) # EUROPEAN PATENT SPECIFICATION

(45) Date of publication of patent specification: **25.01.84**

(51) Int. Cl.³: **C 07 D 501/46,**
A 61 K 31/545
//C07D233/90, C07D487/04

(21) Application number: **80104026.2**

(22) Date of filing: **27.03.80**

(60) Publication number of the earlier application in accordance with Art. 76 EPC: **0019067**

(54) **Cephalosporin derivatives.**

(30) Priority: **27.03.79 JP 36133/79**

(43) Date of publication of application:
**15.04.81 Bulletin 81/15**

(45) Publication of the grant of the patent:
**25.01.84 Bulletin 84/4**

(84) Designated Contracting States:
**CH DE GB IT LI**

(56) References cited:
**FR - A - 2 266 507**
**FR - A - 2 394 550**

(73) Proprietor: **AJINOMOTO CO., INC.**
**5-8, Kyobashi 1-chome, Chuo-ku**
**Tokyo 104 (JP)**

(72) Inventor: **Yasuda, Naohiko**
**2-36-2, Hairando, Yokosuka-shi**
**Kanagawa-ken (JP)**
Inventor: **Eguchi, Chikahiko**
**2136-10, Kamigo-chi, Totsuka-ku**
**Yokohama-shi Kanagawa-ken (JP)**
Inventor: **Okutsu, Masaru**
**3383-18, Fukuda, Yamato-shi**
**Kanagawa-ken (JP)**
Inventor: **Hirose, Yoshiteru**
**1982-106, Fueda, Kamakura-shi**
**Kanagawa-ken (JP)**

(74) Representative: **Schübel-Hopf, Ursula et al,**
**Strehl, Schübel-Hopf, Schulz Patentanwälte**
**Widenmayerstrasse 17**
**D-8000 München 22 (DE)**

**The file contains technical information submitted after the application was filed and not included in this specification**

EP 0 026 811 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Cephalosporin derivatives

The present invention relates to novel imidazoledicarboxylic acid derivatives of cephalosporin, which are useful as antibiotics, particularly as antibacterial agents. They are specifically useful for the treatment of infectious diseases in both human beings and animals, which are caused by Pseudomonas aeruginosa. A new reactive derivative of imidazole dicarboxylic acid useful for producing these cephalosporin derivatives is the subject of the parent application 80 101 662.7.

The present inventors have succeeded in synthesizing novel imidazoledicarboxylic acid derivatives, represented by the formula:

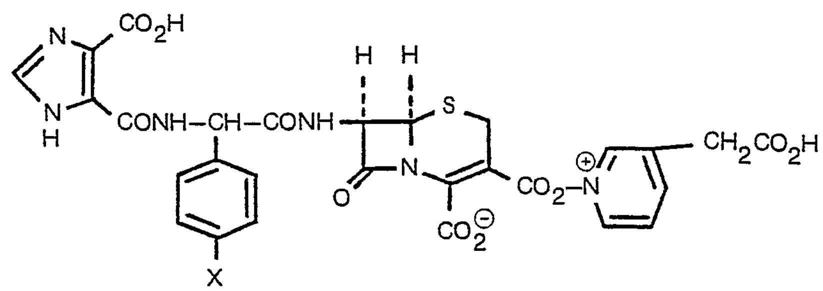

wherein X is a hydrogen atom or a hydroxyl group, and pharmaceutically acceptable nontoxic salts thereof, and have discovered that these novel compounds have a good antibacterial activity, particularly against Pseudomonas aeruginosa and can be used as antibiotics, particularly for oral administration.

The amino acid which forms the compounds of the present invention is phenylglycine, or 4-hydroxyphenylglycine which may be in the L-, D-form, or DL-form. In many cases, the D-form is suitable in view of antibacterial activity. Suitable pharmaceutically acceptable salts of such compounds are nontoxic salts.

The imidazoledicarboxylic acid derivative of the present invention can be prepared by reacting a compound having the formula:

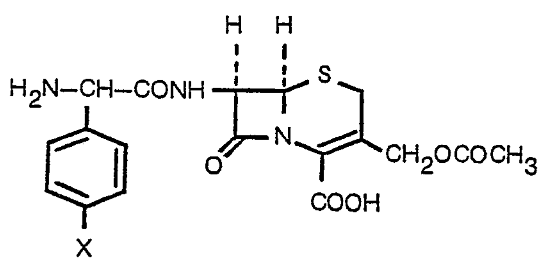

with an imidazoledicarboxylic acid having the formula:

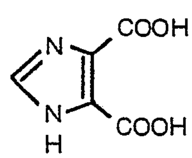

in the form of its reactive derivative such as the acid chloride derivative or with a compound having the formula:

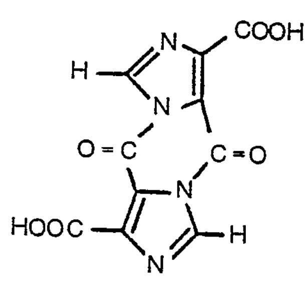

to produce a compound having the formula

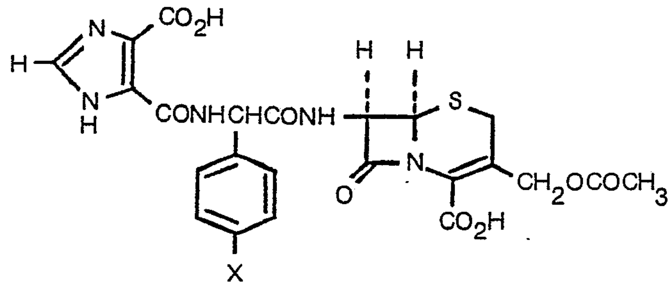

and reacting the compound with 3-carboxymethylpyridine having the formula:

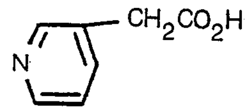

In such formulae, X has the same meaning as above.

The reaction products of the modification reaction can be isolated in pure form by known procedures, for example by extraction, column chromatography and recrystallization. The present invention is explained precisely by the following examples.

Example 1

Imidazoledicarboxylic acid (7.8 g, 0.05 M) was suspended in dry benzene (110 ml), and thereto DMF (4 ml) and then thionyl chloride (30 ml) were added. The thus obtained mixture was refluxed at a temperature of 85°C while stirring. After completion of the reaction, the reaction mixture was concentrated to yield a solid material. To the thus obtained material, dry benzene (50 ml) was added and the mixture was again concentrated to yield a solid material. To the remaining material benzene (50 ml) was added and the mixture was stirred at room temperature for 30 minutes. An insoluble solid material was collected by filtration and then washed with benzene and dried under reduced pressure to give the desired product, 5,10-dioxo-5,10-dihydrodiimidazo [1,5-a,1′,5′-d]pyrazine-1,6-dicarboxylic acid dichloride (7,0 g, yield: 89%).

Elemental analysis:

Found C 38.03%, H 0.74%, N 17.81%, Cl 22.37%

Calcd. as $C_{10}H_2N_4O_4Cl_2$

C 38.36%, H 0.64%, N 17.90%, Cl 22.65%

The thus obtained acid chloride (7.0 g) was suspended in water (150 ml) and the mixture was stirred at 10—20°C overnight. An insoluble solid material was obtained by filtration and washed with water, a small amount of THF, and then ether. The material was dried under reduced pressure to give the desired product, 5,10-dioxo-5,10-dihydrodiimidazo [1,5-a,1′,5′-d]pyrazine-1,6-dicarboxylic acid dihydrate (7.0 g, yield: 100%).

M.P., 284°C (dec.)

I.R. spectrum:

$3500\ cm^{-1}$, $1750\ cm^{-1}$, $1710\ cm^{-1}$, $1255\ cm^{-1}$, $930\ cm^{-1}$

Elemental analysis:

Found C 38.65%, H 2.40%, N 18.02%,

Calcd. as $C_{10}H_4N_4O_6 \cdot 2H_2O$

C 38.47%, H 2.58%, N 17.95%

Anhydrous 7-$\beta$-[D(—)-$\alpha$-aminophenylacetamido]cephalosporanic acid (6.5 g, 16 mM) was suspended in dry dichloromethane (100 ml) and triethylamine (6 ml) was added thereto. The mixture was stirred for 30 minutes while being cooled with ice-water. To this solution 5,10-dioxo-5,10-dihydrodiimidazo[1,5-a,1′,5′-d] pyrazine-1,6-dicarboxylic acid dihydrate (2.2 g, 7 mM) was added while stirring and cooling. The mixture was stirred overnight, and concentrated to give a solid material. This material was added to water (50 ml) and stirred to give a homogenous solution. The solution was adjusted to pH 8 with 6% aqueous HCl solution, stirred for 10 minutes, and then washed with ethyl acetate (50 ml). The water phase was adjusted to pH 2 with 6% aqueous HCl solution, and the mixture was stirred for 20 minutes. The precipitated crystalline material was collected by filtration, washed with water, and then dried under reduced pressure at 40°C. The thus obtained solid material was suspended in a solvent (500 ml) obtained by mixing ethyl acetate and methanol (volume ratio: 1/1), and the mixture was stirred at 40°C for 20 minutes. An insoluble material was separated by filtration. The thus

obtained organic phase was concentrated under reduced pressure to a volume of 50 ml, and ether (500 ml) was added thereto. This mixture was left overnight in the refrigerator. Thus precipitated crystals were collected, washed with petroleum ether and dried to give the desired product, 7-β-[D(—)-α-(4-carboxyimidazole-5-carboxamido)phenylacetamido]cephalosporanic acid, yield: 75%.

7β-[D-(—)-α-(4-carboxyimidazole-5-carboxamido)phenyl-acetamido]cephalosporanic acid. 2Na salt (1,17 g, 2mM) was reacted with 3-pyridylacetic acid (0,55 g, 4 mM) to give 7β-[D-(—)-α-(4-carboxyimidazole-5-carboxamido)phenyl-acetamido]-3-(3-carboxymethylpyridinium) methyl-3-cephem-4-carboxylic acid disodium salt (0,17 g, yield: 12%) in aqueous solution under adjusting the pH to 7 with 2N NaOH solution. To the solution sodium iodide was added (8,3 g) and the solution was reacted at 70°C with stirring for two hours. The reaction mixture was treated with Amberlite®XAD-2 produced by Rohm and Haas Co. in a column. By dilution with water the fractions containing the desired compound were collected. The object compound as above defined was finally obtained by lyophilization of the obtained solution.

I.R. spectrum (Nujol)
$V_{C=O}$ (β-lactam) = 1760 $cm^{-1}$
N.M.R. spectrum ($D_2O$, δ)
ppm 3.35 (m, 2H) (>$CH_2$, 2nd position)

3.60 (s, 2H) 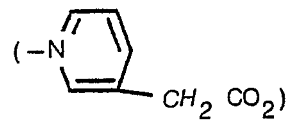

5.10 (d, 1H) (—H, 6th position)

5.60 (s, 1H) 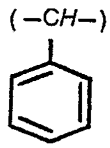

5.73 (d, 1H) (—H, 7th position)

7.45 (m, 5H) 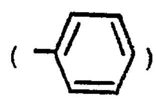

7.80 (s, 1H) (—H, 2nd position in imidazole)

7.90, 8.40 (m, 4H) 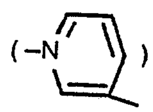

The object compound showed an MIC when using Pseudomonas aeruginosa AJ 1116 as a test organism of 12.5 μg/ml.

## Claims

1. An imidazoledicarboxylic acid derivative having the following formula:

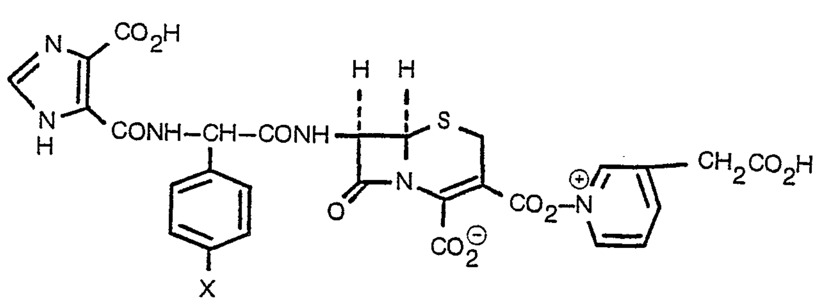

wherein X is a hydrogen atom or a hydroxyl group.

2. An imidazoledicarboxylic acid derivative of Claim 1, wherein the amino acid forming said imidazoledicarboxylic acid derivative is in the D-form.

3. An imidazoledicarboxylic acid derivative of Claim 1, wherein said derivative is in the salt form.

4. An imidazoledicarboxylic acid derivative according to any of the Claims 1 to 3 for the use as an antibacterial agent.

**Patentansprüche**

1. Imidazoldicarbonsäurederivat der Formel

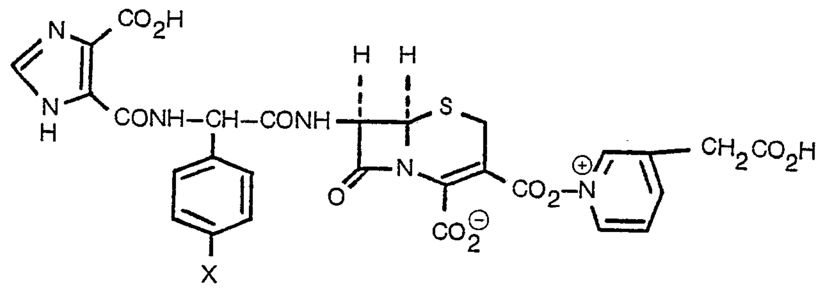

in der X ein Wasserstoffatom oder eine Hydroxylgruppe bedeutet.

2. Imidazoldicarbonsäurederivat nach Anspruch1, in dem die Aminosäure, welche das Imidazoldicarbonsäurederivat bildet, in der D-Form vorliegt.

3. Imidazoldicarbonsäurederivat nach Anspruch 1, wobei das Derivat in der Salzform vorliegt.

4. Imidazoldicarbonsäurederivat nach einem der Ansprüche 1 bis 3 zur Verwendung als antibakterielles Mittel.

**Revendications**

1. Dérivé de l'acide imidazoledicarboxylique de formule:

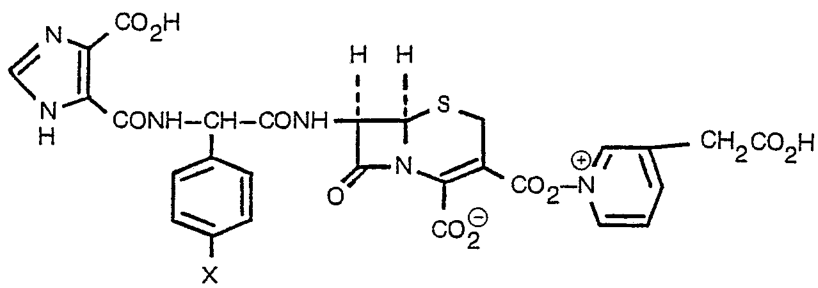

dans laquelle X est un atome d'hydrogène ou un groupe hydroxyle.

2. Dérivé de l'acide imidazoledicarboxylique selon la revendication 1, où l'amino-acide formant le dérivé de l'acide imidazoledicarboxylique est en forme D.

3. Dérivé de l'acide imidazoledicarboxylique selon la revendication 1, où le dérivé est en forme de sel.

4. Dérivé de l'acide imidazoledicarboxylique selon une des revendications 1 à 3 pour l'emplois comme agent antibacteriel.